# EUROPÄISCHE PATENTANMELDUNG

(11) **EP 4 717 793 A2**
(43) Veröffentlichungstag der Anmeldung: **01.04.2026**
(21) Anmeldenummer: 26159189.5
(22) Anmeldetag: 21.03.2022
(51) Int. Cl.: C23C 8/00

(54) **CHIRURGISCHES ODER ENDOSKOPISCHES INSTRUMENT UND DESSEN HERSTELLUNG**

(30) Priorität: 23.03.2021 DE 102021107219
(62) Teilanmeldung aus: 22718076.7
(71) Anmelder: Karl Storz SE & Co. KG, 78532 Tuttlingen (DE)
(72) Erfinder: FOMIN, Anton, 78532 Tuttlingen (DE); BRAUNSCHWEIG, Dominik, 78532 Tuttlingen (DE); SCHWENDER, Anna, 78532 Tuttlingen (DE); KÖNIG-URBAN, Kamilla, 78532 Tuttlingen (DE)

(57) **Zusammenfassung**

Ein chirurgisches oder endoskopisches Instrument ist mit zumindest einem Längsglied (28, 30; 128; 228, 230; 328) mit einer Längserstreckung zwischen einem ersten Ende und einem zweiten Ende, und einem Trägerstück (50, 52; 150, 152; 250, 252; 350, 352; 410; 412; 414; 416) versehen, das zumindest einen strukturierten Kontaktabschnitt (60, 62; 160, 162; 260, 262; 360, 362; 420; 422; 424; 426) aufweist, der insbesondere mit einer Verzahnung oder Riffelung versehen ist, wobei der zumindest eine strukturierte Kontaktabschnitt (60, 62; 160, 162; 260, 262; 360, 362; 420; 422; 424; 426) in das Trägerstück (50, 52; 150, 152; 250, 252; 350, 352; 410; 412; 414; 416) integriert ist, wobei das Trägerstück (50, 52; 150, 152; 250, 252; 350, 352; 410; 412; 414; 416) und der zumindest eine strukturierte Kontaktabschnitt (60, 62; 160, 162; 260, 262; 360, 362; 420; 422; 424; 426) aus einem korrosionsbeständigen Stahlwerkstoff bestehen, insbesondere aus einem Edelstahl, und wobei der zumindest eine strukturierte Kontaktabschnitt (60, 62; 160, 162; 260, 262; 360, 362; 420; 422; 424; 426) oberflächennah niedertemperatur-diffusionsgehärtet ist. Ferner bezieht sich die Offenbarung auf ein Verfahren zur Herstellung eines chirurgischen oder endoskopischen Instruments.

## Beschreibung

Die vorliegende Offenbarung bezieht sich auf medizinische Instrumente, insbesondere chirurgische oder endoskopische Instrumente, sowie auf ein Verfahren zu deren Herstellung. Insbesondere bezieht sich die Offenbarung auf Instrumente mit erhöhter Härte im Bereich der Kontaktflächen, mit denen das Instrument mit Gewebe, Organen oder medizinischen Gerätschaften interagiert.

Aus der WO 2020/004667 A1 ist ein miniaturisiertes Instrument in Form einer okularen Pinzette oder Zange für die Augenchirurgie bekannt. Das Instrument weist einen durchmesserkleinen Schaft auf, an dessen distalem Ende ein miniaturisierter Endeffektor mit Stoffgelenk ausgebildet ist. Der Schaft sitzt in einer dünnen Hülse, die sich entlang der Längserstreckung des durchmesserkleinen Schafts zwischen einem proximalen Ende des Schafts und dem distalen Ende erstreckt, wobei am proximalen Ende ein Handgriff zur Betätigung vorgesehen ist, der die Hülse relativ zum Schaft nach proximal oder nach distal bewegt, wobei die Hülse am distalen Ende auf den miniaturisierten Endeffektor einwirkt, um diesen zu öffnen oder zu schließen.

Aus der US 6,077,280 A ist ein Maulteilinstrument mit strukturierten Greifbacken bekannt, das zur Entfernung von Fremdkörpern aus dem menschlichen Körper dient. Zum Greifen und Halten der Fremdkörper weist das Instrument Maulteile mit sogenannten Rattenzähnen auf, deren Spitzen und Lücken aneinander angepasst sind, so dass einander gegenüberliegende Rattenzähne der Maulteile im geschlossenen Zustand ineinandergreifen können, um Fremdkörper zu sichern.

Aus der US 6,322,578 B1 ist ein Instrument in Form eines Nadelhalters bekannt, wobei das Instrument an seinem distalen Ende zwei Maulteile aufweist. Es wird vorgeschlagen, dort separate Einsätze aus gehärtetem Metall vorzusehen, damit die Maulteile chirurgische Nadeln und dergleichen greifen können. Aus der WO 2018/053223 A1 ist ein Ultraschallinstrument bekannt, das an seinem distalen Ende einen Bearbeitungskopf mit Verzahnung aufweist. Das Instrument dient zum Abtragen von Hartgewebe oder Knochengewebe. Aus der WO 2016/134030 A2 ist eine chirurgische Säge in Form eines Oszillationswerkzeugs bekannt, wobei ein Sägeblatt vorgesehen ist, das aus einem Edelstahlwerkstoff besteht.

Das Merkblatt "Oberflächenhärten nichtrostender Stähle" (N.N.: Merkblatt 982 Oberflächenhärten nichtrostender Stähle, Informationsstelle Edelstahl Rostfrei; Düsseldorf 2015; ISBN 978-2-87997-069-1) beschreibt verschiedene Härteverfahren für nichtrostende Stähle. Die US 5 342 365 A beschreibt eine chirurgische Raspel mit Arbeitseinsätzen aus Wolframkarbid. Die DE 10 2009 005 578 A1 beschreibt chirurgische Instrumente aus einem ferritischen Chromstahl, die durch eine Wärmebehandlung eine erhöhte Härte in der Randschicht erhalten. Die EP 2 543 326 A1 beschreibt Bohrwerkzeuge für Knochenbohrer, wobei das Bohrwerkzeug aus rostfreiem Stahl mit einem Mindestanteil von 40 % Austenit besteht und einer Oberflächenhärte von mindestens 850 HV 0,05 aufweist, die durch das Vorhandensein von Druckspannungen entsteht, die durch die interstitielle Auflösung der Kohlenstoffatome im Austenit entstehen.

Medizinische Instrumente, insbesondere chirurgische oder endoskopische Instrumente sind hinreichend bekannt. Beispielhaft kann es sich um medizinische Zangen, Nadelhalter, Pinzetten, Feilen, Raspeln und ähnliche Instrumente handeln.

Es gibt medizinische Instrumente, die üblicherweise mit weichem Gewebe oder anderen weichen Materialien zusammenwirken.

Es gibt jedoch auch medizinische Instrumente, die mit härteren Oberflächen und Teilen konfrontiert werden. Dies betrifft zum Beispiel sogenannte Nadelhalter zum Führen von Nadeln beim chirurgischen Nähen. Ferner betrifft dies zum Beispiel medizinische Raspeln und Feilen (beispielsweise Knochenraspeln und Knochenfeilen), die zum Abtragen und Bearbeiten von Knochen und hartem Gewebe verwendet werden. Derartige Instrumente brauchen also Kontaktflächen und Kontaktabschnitte mit entsprechender Härte, die nicht ohne weiteres durch die genannten harten Materialien verschleißen oder gar abgetragen werden.

Dies gilt umso mehr, wenn die Instrumente im Bereich ihrer Funktionsflächen (Kontaktabschnitte) mit einer Strukturierung versehen sind, etwa in Form einer Verzahnung oder Riffelung. Eine derartige Strukturierung dient etwa bei Raspeln oder Feilen dem Materialabtrag. Bei Nadelhaltern, Pinzetten und ähnlichen Instrumenten, die zum Greifen geeignet sind, sorgt eine Strukturierung für einen festen und sicheren Griff.

Medizinische Instrumente sollen grundsätzlich biokompatibel und korrosionsbeständig sein. Daher werden medizinische Instrumente der eingangs genannten Art häufig aus korrosionsarmen oder korrosionsfreien Stählen, insbesondere aus rostfreiem Edelstahl hergestellt. Dies minimiert einerseits Reaktionen mit dem Körper (des Patienten). Andererseits erlauben derartige Werkstoffe die (wiederholte) Reinigung und Sterilisation.

Häufig ist auch eine gewisse Elastizität bzw. Duktilität (Zähigkeit) gewünscht, weil die medizinischen Instrumente im Einsatz häufig stark belastet und gegebenenfalls sogar verformt werden.

Diese gegenläufigen Forderungen nach harten Abschnitten zum Greifen und gleichwohl einer hinreichenden Elastizität und Zähigkeit führen dazu, dass Nadelhalter und ähnliche Instrumente mit hohen Anforderungen an die Oberflächenhärte regelmäßig Backen/Platten aus einem anderen Werkstoff mit hoher Härte aufweisen, der auf einen Träger (üblicherweise Edelstahl) appliziert ist. Regelmäßig werden sogenannte Hartmetallplatten oder Hartmetallbacken verbaut, die beispielsweise stoffschlüssig (Löten, Schweißen) mit dem Trägermaterial verbunden werden.

Auf diese Weise kann einerseits der gewünschte Kompromiss aus Oberflächenhärte und Zähigkeit erreicht werden. Jedoch sind Hartmetallteile hinsichtlich der Biokompatibilität nicht vollkommen unproblematisch.

Ferner hat sich gezeigt, dass das Fügen der Hartmetallteile mit dem Trägermaterial (üblicherweise korrosionsarmer Stahl oder Edelstahl) zu optischen Beeinträchtigungen führen kann, die nicht immer vom Markt akzeptiert werden.

Bei strukturierten Teilen aus Hartmetall, also beispielsweise mit verzahnten oder geriffelten Kontaktflächen, ist zu beachten, dass einzelne Segmente (Zähne) zwar sehr hart sind, aber daher unter Umständen auch brechen können.

Bei einem Verbund aus Edelstahl und Hartmetall muss beim Betrieb und bei der Reinigung/Sterilisation darauf geachtet werden, dass unterschiedliche Wärmeausdehnungskoeffizienten vorliegen, so dass gegebenenfalls Spannungen an der Grenze zwischen Edelstahl und Hartmetall entstehen. Dies kann so weit gehen, dass sich Hartmetallplatten von ihrem Trägermaterial lösen. Aus diesem Grund muss der Fügevorgang zwischen Hartmetall und Trägermaterial mit großer Sorgfalt ausgeführt werden. Dies erhöht die Herstellkosten.

Der vorliegenden Offenbarung liegt daher die Aufgabe zugrunde, medizinische Instrumente, insbesondere chirurgische oder endoskopische Instrumente, anzugeben, die hinsichtlich der Biokompatibilität und hinsichtlich der erzielbaren Oberflächenhärte günstige Eigenschaften aufweisen.

Vorzugsweise kann eine oberflächennahe Randzone mit guter Oberflächenhärte geschaffen und gleichzeitig die gewünschte Zähigkeit/Duktilität im Kern gewährleistet werden.

Derartige Instrumente sollen möglichst mit geringem Aufwand herstellbar sein. Ferner sollen die Instrumente eine optisch möglichst integrale Gestaltung aufweisen. Die Instrumente sollen möglichst verschleißarm eingesetzt werden können und eine hohe Lebensdauer und Belastbarkeit aufweisen.

Gemäß einem ersten Aspekt bezieht sich die vorliegende Offenbarung auf ein als Maulkopfinstrument gestaltetes chirurgisches oder endoskopisches Instrument, mit zumindest einem Längsglied mit einer Längserstreckung zwischen einem ersten Ende und einem zweiten Ende, und mit zwei Maulteilen, die relativ zueinander bewegbar sind, wobei jedes der zwei Maulteile ein Trägerstück ausbildet, das zumindest einen strukturierten Kontaktabschnitt aufweist, der insbesondere mit einer Verzahnung oder Riffelung versehen ist, wobei die strukturierten Kontaktabschnitte der zwei Maulteile zumindest im geschlossenen Zustand einander zugewandt sind, wobei die strukturierten Kontaktabschnitte in das jeweilige Trägerstück integriert sind, wobei das jeweilige Trägerstück und der jeweilige strukturierte Kontaktabschnitt aus einem korrosionsbeständigen Stahlwerkstoff bestehen, nämlich aus einem Edelstahl, und wobei die strukturierten Kontaktabschnitte oberflächennah niedertemperatur-diffusionsgehärtet sind, nämlich durch eine Oberflächenbehandlung mittels Niedertemperatur-Diffusionshärten unter Ausbildung einer oberflächennahen Diffusionszone.

Die Aufgabe der Offenbarung wird auf diese Weise gelöst.

Offenbarungsgemäß lassen sich auf diese Weise hinreichend biokompatibel gestaltete medizinische Instrumente bereitstellen, die gleichwohl im Bereich ihrer strukturierten Kontaktabschnitte eine ausgezeichnete Oberflächenhärte aufweisen.

Auf diese Weise sind die Instrumente mechanisch hochbelastbar. Gleichzeitig ist eine gute Verträglichkeit mit Gewebe gewährleistet. Daneben eignen sich die Instrumente für intensive Reinigungsvorgänge und insbesondere für die Sterilisation.

Vorzugsweise wird auf Hartmetallwerkstoffe verzichtet, ohne jedoch auf deren günstige mechanische Eigenschaften verzichten zu müssen, insbesondere auf die erzielbare Härte im Bereich der strukturierten Kontaktabschnitte.

In einer beispielhaften Ausgestaltung weist die Strukturierung beispielsweise eine oder zwei Vorzugsrichtungen auf, dies ist jedoch nicht einschränkend zu verstehen. Die Strukturierung kann ähnlich einem Rändel oder Kreuzrändel gestaltet sein. Die Strukturierung kann jedoch auch einzelne Zähne aufweisen, die beispielsweise in Reihen mit einer Vorzugsrichtung oder mit zwei oder mehr Vorzugsrichtungen angeordnet sind. Eine Riffelung kann etwa längliche Kerben umfassen, die in einer oder zwei Vorzugsrichtungen angeordnet sind. Bei zwei Vorzugsrichtungen können sich die Kerben kreuzen.

Die Instrumente sind korrosionsbeständig und vorzugsweise rostfrei gestaltet, unter Berücksichtigung der üblichen Anwendungsbedingungen für medizinische Instrumente.

Das Trägerstück mit dem strukturierten Kontaktabschnitt kann auch als Backe oder Backen bezeichnet werden, zumindest in beispielhaften Ausgestaltungen.

Das Trägerstück und der strukturierte Kontaktabschnitt sind aus ein und demselben Werkstoff integral gefertigt. Mit anderen Worten bildet der strukturierte Kontaktabschnitt einen integralen Bestandteil des Trägerstücks. Es handelt sich also nicht um eine separate Platte oder dergleichen, die durch ein Fügeverfahren mit dem Trägerstück verbunden wird.

Es versteht sich, dass sich die Begriffe chirurgisch und endoskopisch nicht gegenseitig ausschließen müssen, zumindest in beispielhaften Ausgestaltungen. Es sind endoskopische chirurgische Instrumente bekannt. Allgemein handelt es sich um medizinische Instrumente, die körpernah oder im Körper eingesetzt werden können. Ein offenbarungsgemäßes medizinisches Instrument weist beispielhaft eine ausgeprägte Längserstreckung zwischen einem proximalen Ende und einem distalen Ende auf. Entlang der Längserstreckung erstrecken sich ein oder mehrere Längsglieder.

Im Sinne der vorliegenden Offenbarung ist das distale Ende üblicherweise ein vom Bediener abgewandtes Ende, das dem Patienten zugewandt ist. Im Sinne der vorliegenden Offenbarung ist das proximale Ende üblicherweise ein dem Bediener zugewandtes Ende, das vom Patienten abgewandt ist. Das distale Ende kann auch als patientennahes Ende bezeichnet werden. Das proximale Ende kann auch als patientenfernes Ende bezeichnet werden. Dies ist jedoch nicht einschränkend zu verstehen.

Die Härtesteigerung an der Oberfläche wird unter Verzicht auf eine Beschichtung ermöglicht. Dies umfasst beispielsweise den Verzicht auf eine Diamantbeschichtung oder eine ähnliche Beschichtung mit harten Schneidstoffen oder anderen härtesteigernden Stoffen.

Das Trägerstück und der strukturierte Kontaktabschnitt bestehen aus dem gleichen Werkstoff und sind insbesondere integral gefertigt. Es handelt sich um austenitische, rostfreie Edelstähle, zumindest in beispielhaften Ausgestaltungen.

Austenitische, rostfreie Edelstähle besitzen regelmäßig eine hohe Korrosionsbeständigkeit gegen Wasser und diverse Chemikalien. Jedoch sind die Härte und - damit verknüpft - die Verschleißfestigkeit der Oberflächen nur begrenzt. Dies zeigt sich insbesondere beim unmittelbaren Kontakt mit harten Objekten. Das Härten solcher Edelstähle mit herkömmlichen Wärmebehandlungsverfahren ist nicht ohne weiteres möglich. Zumindest muss mit nachteiligen Auswirkungen auf die Korrosionsbeständigkeit und gegebenenfalls die optische Erscheinung (Farbgebung) gerechnet werden.

Gemäß der vorliegenden Offenbarung erfolgt die härtesteigernde Oberflächenbehandlung durch ein Niedertemperatur-Diffusionshärten, das insbesondere als Niedertemperatur-Kohlenstoffdiffusionsverfahren gestaltet ist. Vergleichbare Niedertemperatur-Diffusionsverfahren zur Härtesteigerung sind als Stickstoffdiffusionsverfahren gestaltet und werden von der vorliegenden Offenbarung umfasst.

In beispielhaften Ausgestaltungen erfolgt die Oberflächenbehandlung gemäß dem sogenannten Kolsterisieren (eine zum Zeitpunkt der Hinterlegung dieser Offenbarung eingetragene deutsche Marke der Bodycote plc, Macclesfield Cheshire, Großbritannien).

Niedertemperatur-Diffusionshärteverfahren umfassen beispielsweise eine Härtung der Randschicht bei weniger als 500 °C. Bei der Härtung können beträchtliche Mengen des Kohlenstoffs in die Diffusionszone eindiffundiert werden. Der Kohlenstoff wird dabei in Zwischengitterplätzen gelöst. Die Bildung von Karbiden wird vermieden. Im Ergebnis führt der eindiffundierte Kohlenstoff zu erhöhten Druckspannungen im oberflächennahen Bereich, so dass insgesamt eine günstige, deutlich gegenüber dem Ursprungszustand erhöhte Oberflächenhärte entsteht.

Weitere Vorteile des Niedertemperatur-Diffusionshärtens sind eine günstige Formstabilität und maßliche Stabilität. Ferner können gerade bei austenitischen Edelstählen ungünstige Farbveränderungen vermieden werden, welche bei konventionellen Härteverfahren gegebenenfalls die optische Wahrnehmung beeinträchtigen würden.

Offenbarungsgemäße Instrumente tragen dazu bei, Hartmetalle bei medizinischen Anwendungen weiter zu substituieren. Zumindest kann der Anteil von Hartmetallen und anderen Fremdstoffen (zum Beispiel Diamant, kubisches Bornitrid und ähnliche Hartstoffe) weiter zurückgedrängt werden.

Vorzugsweise bleibt dabei die Korrosionsbeständigkeit erhalten. Die Biokompatibilität verbessert sich zumindest durch den Wegfall potenziell problematischer Werkstoffe.

Gemäß einem weiteren Aspekt bezieht sich die vorliegende Offenbarung auf ein Verfahren zur Herstellung eines chirurgischen oder endoskopischen Instruments gemäß zumindest einer der hierin beschriebenen Ausgestaltungen, wobei das Verfahren die folgenden Schritte aufweist:
- Bereitstellung zumindest eines Längsgliedes mit einer Längserstreckung zwischen einem ersten Ende und einem zweiten Ende,
- Bereitstellung eines Trägerstückes aus einem korrosionsbeständigen Stahlwerkstoff, insbesondere aus einem Edelstahl, wobei das Trägerstück als Abschnitt des zumindest einen Längsgliedes oder als mit dem Längsglied gekoppeltes Teil gestaltet ist,
- Erzeugung zumindest eines strukturierten Kontaktabschnitts auf dem Trägerstück, insbesondere umfassend Erzeugung einer Verzahnung oder Riffelung, und
- Durchführung einer lokalen Oberflächenbehandlung mittels Niedertemperatur-Diffusionshärten unter Ausbildung einer oberflächennahen Diffusionszone zum Härten des zumindest einen strukturierten Kontaktabschnitts.

Auch auf diese Weise wird die Aufgabe der Offenbarung gelöst.

Das offenbarungsgemäße Verfahren zur Herstellung medizinischer Instrumente kann analog zu den hierin gezeigten Ausgestaltungen und Ausführungsformen der chirurgischen oder endoskopischen Instrumente weitergebildet sein. Gleichermaßen können die offenbarungsgemäßen Instrumente analog zu Ausführungsformen des offenbarungsgemäßen Verfahrens weitergebildet sein.

Insbesondere können offenbarungsgemäße Instrumente unter Nutzung des offenbarungsgemäßen Herstellverfahrens hergestellt werden. Das offenbarungsgemäße Verfahren eignet sich zur Herstellung und Wärmebehandlung offenbarungsgemäßer medizinischer Instrumente.

Gemäß einer beispielhaften Ausgestaltung des Instruments oder des Verfahrens bestehen das Trägerstück und der zumindest eine strukturierte Kontaktabschnitt aus einem biokompatiblen Stahlwerkstoff, insbesondere aus einem biokompatiblen Edelstahl.

Im Sinne der vorliegenden Offenbarung ist unter dem Begriff Biokompatibilität die Fähigkeit des Instruments zu verstehen, während der Anwendung nachteilige Wechselwirkungen mit dem behandelten Körper zu vermeiden. Edelstähle werden im Allgemeinen als hinreichend biokompatibel zur Verwendung bei chirurgischen oder endoskopischen Instrumenten erachtet.

Zumindest in beispielhaften Ausgestaltungen ist der Begriff Biokompatibilität im Sinne der EU-Medizinprodukteverordnung (Verordnung (EU) 2017/745) zu verstehen. Beispielhaft erläutert unter dieser Maßgabe die Norm ISO-10993 (Ausgabe DIN EN ISO 10993-1:2010-04) den Begriff Biokompatibilität unter Berücksichtigung der chirurgischen oder endoskopischen Anwendung.

Das Instrument ist beispielsweise als Nadelhalter gestaltet. Das Instrument ist beispielsweise als medizinische Pinzette gestaltet. Gemäß weiteren alternativen Ausgestaltungen des Instruments oder des Verfahrens ist das Instrument ausgewählt aus der Gruppe, die aus Folgendem besteht: medizinische Feilen und medizinische Raspeln.

Insbesondere handelt es sich um medizinische Instrumente, die im Einsatz mit hinreichend harten Partnern (Werkstoffe, Gewebe und dergleichen) interagieren bzw. auf diese einwirken. Dies umfasst beispielsweise hartes Gewebe wie Knochen, Knorpel und dergleichen. Ferner kann dies auch Medizinprodukte aus hinreichend harten Werkstoffen wie chirurgische Nadeln, Kanülen und Ähnliches betreffen.

Beispielsweise dienen medizinische Feilen oder medizinische Raspeln zum gezielten Abtrag von Knochen, Knorpeln und sonstigem harten Gewebe.

Beispielsweise dienen Nadelhalter zum Greifen, Halten und Handhaben chirurgischer Nadeln.

Diese und ähnliche medizinische Instrumente profitieren von härtesteigenden Maßnahmen im Bereich der jeweiligen Kontaktflächen, die mit den harten Partnern in Kontakt treten.

Gemäß einer weiteren beispielhaften Ausgestaltung des Instruments oder des Verfahrens sind zumindest das Trägerstück und der zumindest eine strukturierte Kontaktabschnitt, vorzugsweise das gesamte Instrument, hartmetallfrei gestaltet.

Gleichwohl ist es wünschenswert, wenn der strukturierte Kontaktabschnitt Eigenschaften aufweist, die insbesondere hinsichtlich der Oberflächenhärte Hartmetallen vergleichbar sind. Mit anderen Worten kann bei dem Instrument auf Hartmetallplättchen zur Ausbildung des strukturierten Kontaktabschnitts verzichtet werden.

Gemäß einer weiteren beispielhaften Ausgestaltung des Instruments oder des Verfahrens weist der zumindest eine strukturierte Kontaktabschnitt eine gesteigerte Oberflächenhärte auf.

Vorzugsweise erfolgt der Härtevorgang derart, dass primär derjenige Bereich, der bei der Anwendung tatsächlich hochbelastet ist, einer härtesteigernden Behandlung unterzogen wird. Andere Bereiche müssen keiner solchen Behandlung unterzogen werden.

Gemäß einer weiteren beispielhaften Ausgestaltung des Instruments oder des Verfahrens weist der zumindest eine strukturierte Kontaktabschnitt eine Oberflächenhärte nach Vickers von zumindest 750 HV 0.05 auf, gemäß einer weiteren beispielhaften Ausgestaltung zumindest 850 HV 0.05, gemäß einer weiteren beispielhaften Ausgestaltung zumindest 950 HV 0.05, gemäß einer weiteren beispielhaften Ausgestaltung zumindest 1050 HV 0.05. Dies gilt für den Zustand nach der Durchführung der härtesteigernden Oberflächenbehandlung.

Auf diese Weise eignet sich das Instrument für verschiedene Anwendungen, bei denen es auf eine gesteigerte Oberflächenhärte ankommt, beispielsweise als Nadelhalter, medizinische Feile oder Raspel, und dgl.

Die Härteprüfung nach Vickers mit der Prüfbedingung HV 0.05 geht beispielsweise einher mit einer Prüfkraft F in Höhe von etwa 0,4903 N (Newton). Es handelt sich bei der Härteprüfung HV 0.05 um eine Mikrohärteprüfung. Die Härteprüfung nach Vickers eignet sich für oberflächengehärtete Werkstücke. Die Härteprüfung nach Vickers erfolgt beispielsweise nach der Maßgabe der Norm DIN EN ISO 6507-1:2018 bis DIN EN ISO 6507-4:2018.

Je nach konkreter Ausprägung lassen sich durch das Niedertemperatur-Diffusionshärten maximale Oberflächenhärten im Bereich von 900 und 1300 HV 0.05 erzeugen, wobei die duktilen Eigenschaften des Kerns grundsätzlich erhalten bleiben.

Gemäß einer weiteren beispielhaften Ausgestaltung des Instruments oder des Verfahrens beträgt die Tiefe der Diffusionszone im strukturierten Kontaktabschnitt 10 µm bis 60 µm (Mikrometer), gemäß einer weiteren beispielhaften Ausgestaltung 25 µm bis 50 µm. Auf diese Weise wird gewährleistet, dass der Kern seine duktilen Eigenschaften bewahrt. Dies reduziert die Bruchanfälligkeit des Instruments.

Gemäß einer weiteren beispielhaften Ausgestaltung des Instruments oder des Verfahrens weist zumindest das Trägerstück einen duktilen Kern auf. Im Kern des Trägerstücks weist also der Basiswerkstoff seine ursprünglichen Eigenschaften auf, es ist eine gute Zähigkeit gegeben. Die Bruchgefahr ist gering.

Gemäß einer weiteren beispielhaften Ausgestaltung des Instruments oder des Verfahrens ist der korrosionsbeständige Stahlwerkstoff aus der Gruppe ausgewählt, die aus Folgendem besteht: nichtrostende, austenitische Chrom-Nickel-Stähle mit niedrigem Kohlenstoffgehalt, nichtrostende, austenitische Chrom-Nickel-Molybdän Edelstähle mit niedrigem Kohlenstoffgehalt, und korrosionsbeständige, warmfeste Eisen-Nickel-Chrom-Stähle.

Ein Beispiel für einen nichtrostenden, austenitischen Chrom-Nickel-Stahl mit niedrigem Kohlenstoffgehalt ist die Werkstoffnummer 1.4307. Beispiele für einen nichtrostenden, austenitischen Chrom-Nickel-Molybdän Edelstahl mit niedrigem Kohlenstoffgehalt sind die Werkstoffnummer 1.4435 und die Werkstoffnummer 1.4404. Ein Beispiel für einen Stahl aus einer korrosionsbeständigen, warmfesten Eisen-Nickel-Chrom-Legierung ist die Werkstoffnummer 1.4980.

Gemäß einer weiteren beispielhaften Ausgestaltung des Instruments oder des Verfahrens ist die Prozesstemperatur des Niedertemperatur-Diffusionsverfahrens unterhalb der Rekristallisationstemperatur des Stahlwerkstoffs gewählt.

Ein Vorteil des offenbarungsgemäßen Niedertemperatur-Diffusionsverfahrens ist eine sehr homogene, ausscheidungsarme oder ausscheidungsfreie Diffusionszone, zumindest in beispielhaften Ausgestaltungen. Auf diese Weise bleiben die günstigen Korrosionseigenschaften der Grundwerkstoffe gewahrt. Die Diffusionszone und die sich anschließende Kernzone sind hinreichend deutlich voneinander abgegrenzt, zumindest in beispielhaften Ausgestaltungen. Dies lässt sich beispielsweise durch metallografische Untersuchungen und vergleichbare Gefügeuntersuchungen zeigen.

Bei konventionellen Wärmebehandlungsverfahren (beispielsweise Nitrocarburieren) sorgen Ausscheidungen dafür, dass die Trennung zwischen Diffusionszone und Kernzone nicht so deutlich ausgeprägt ist. Auf diese Weise ergibt sich bei konventionellen Wärmebehandlungsverfahren eine verringerte Korrosionsbeständigkeit.

Gemäß einer weiteren beispielhaften Ausgestaltung des Instruments oder des Verfahrens ist das Instrument als Maulkopfinstrument gestaltet, insbesondere als Nadelhalter, wobei zwei Maulteile vorgesehen sind, die relativ zueinander bewegbar sind, wobei jedes der zwei Maulteile ein Trägerstück ausbildet und mit einem strukturierten Kontaktabschnitt versehen ist, und wobei die strukturierten Kontaktabschnitte der zwei Maulteile zumindest im geschlossenen Zustand einander zugewandt sind.

Nadelhalter sind spezifische chirurgische Instrumente, die etwa nach Art einer Zange oder Schere gestaltet sind, wobei zwei Maulteile vorgesehen sind, die beim chirurgischen Nähen die Nadel mit entsprechender Haltekraft halten sollen. Aus diesem Grund weisen die Kontaktabschnitte regelmäßig eine Strukturierung auf. Ferner sollten die Kontaktabschnitte eine gewisse Oberflächenhärte bereitstellen. Medizinische Nadeln weisen ihrerseits eine gewisse Härte auf. Medizinische Nadeln und Ähnliches sollen fest und sicher gehalten werden. Demgemäß wirken hohe Klemmkräfte auf die feinen, dünnen Nadeln und die Kontaktabschnitte beim Instrument.

Chirurgische Nähnadeln weisen häufig spezielle Gestaltungen auf, um möglichst atraumatisch (Vermeidung von Gewebeverletzungen) eingesetzt werden zu können. Dies bezieht sich einerseits auf den Durchmesser (das Kaliber). Ferner sind unterschiedliche Formen vorstellbar, beispielsweise gerade Form, leicht gekrümmte Form, stark gekrümmte Form und Ähnliches. Es wird ferner darauf abgestellt, dass möglichst kein Durchmessersprung zwischen der Nadel und dem Faden gegeben ist. Derart spezielle Nadeln können in der Praxis im Regelfall nicht von Hand gehalten und geführt werden. Daher sind Nadelhalter verschiedener Art bekannt. Die Nadelhalter bzw. deren Maulteile sind spezifisch an die verwendeten Nadeln angepasst, zumindest in beispielhaften Ausgestaltungen.

Es versteht sich, dass auch andere Anwendungen als Nähen vorstellbar sind, beispielsweise das Setzen von Knoten.

Es versteht sich, dass auch andere Maulkopfinstrumente vorstellbar sind, die bestimmte Anforderungen an die Oberflächenhärte im Bereich der Backen (mit den strukturierten Kontaktabschnitten) aufweisen. Dies betrifft beispielhaft Instrumente, deren Aufgabe es ist, andere medizinische Instrumente/Utensilien zu halten und zu führen.

Konventionelle Nadelhalter weisen üblicherweise Backen aus Hartmetallwerkstoffen auf, beispielsweise sogenannte Sintermetallbacken. Diese stellen die gewünschte Härte bereit, müssen aber auf die Maulteile (Trägerstücke) appliziert werden. Dies erfolgt beispielsweise mittels Schweißen oder Löten. Aus Sicht der Biokompatibilität ist es wünschenswert, diese Hartmetallteile zu ersetzen.

Die Maulteile können zumindest zwischen einem geschlossenen Zustand und einem geöffneten Zustand relativ zueinander bewegt werden. Im geschlossenen Zustand können beispielsweise eine chirurgische Nadel oder andere chirurgische Gerätschaften fest und sicher gehalten und geführt werden.

Gemäß einer weiteren beispielhaften Ausgestaltung des Instruments oder des Verfahrens ist das Längsglied als Schaft gestaltet, der sich zwischen einem proximalen Ende und einem distalen Ende erstreckt, wobei beim proximalen Ende ein Griffabschnitt angeordnet ist, und wobei beim distalen Ende die zwei Maulteile angeordnet sind, von denen zumindest ein Maulteil relativ zum anderen Maulteil beweglich, insbesondere verschwenkbar, ist.

Ein derart gestaltetes Instrument eignet sich zum Beispiel für endoskopische oder laparoskopische Anwendungen, also zur Anwendung im Körperinneren. Je nach Gestaltung ist gleichwohl auch eine Eignung zur Behandlung des Körpers von außerhalb des Körpers gegeben.

Gemäß einer weiteren beispielhaften Ausgestaltung des Instruments oder des Verfahrens erstreckt sich durch den Schaft zumindest ein Betätigungselement zur Betätigung des zumindest einen beweglichen Maulteils. Auf diese Weise kann das Instrument beispielsweise durch ein Handstück mit zumindest einer Handhabe beim proximalen Ende gehalten und gesteuert werden, ohne dass sich der Schaft beim Öffnen und Schließen der Maulteile notwendigerweise bewegt. Eine solche Gestaltung eignet sich für laparoskopische und endoskopische Instrumente, deren distales Ende in den Körper eingeführt wird.

Gemäß einer weiteren beispielhaften Ausgestaltung des Instruments oder des Verfahrens ist eine Arretierung vorgesehen, die einen geschlossenen Zustand der zwei Maulteile sichert. Auf diese Weise wird sichergestellt, dass beispielsweise bei einem Nadelhalter eine fixierte Nadel oder dergleichen sicher gehalten wird.

Gemäß einer weiteren beispielhaften Ausgestaltung des Instruments oder des Verfahrens ist das Instrument als chirurgische Feile oder Raspel gestaltet, wobei das Längsglied stangenartig gestaltet ist, wobei das zumindest eine Trägerstück als Blatt an einem ersten Ende oder zweiten Ende des Längsgliedes angeordnet ist, und wobei das Blatt zumindest an einer Seite mit einem strukturierten Kontaktabschnitt versehen ist.

Chirurgische Feilen oder Raspeln können beispielsweise zur Bearbeitung von Knochen, Knorpeln oder ähnlichem festen Gewebe verwendet werden. Daher ist auch hier eine erhöhte Oberflächenhärte von Vorteil.

Der Übergang zwischen Feilen und Raspeln ist fließend. Im Sinne einer Definition werden als Raspel im Allgemeinen solche Instrumente bezeichnet, deren Verzahnung aus einzelnen Zähnen gebildet ist, die in bestimmter Anordnung willkürlich oder geordnet positioniert sind. Im Sinne einer Definition werden als Feilen im Allgemeinen solche Instrumente bezeichnet, der Verzahnung durch Reihen durchgehender (gegebenenfalls gekreuzter) Linien gebildet ist. Dies ist nicht einschränkend zu verstehen. Verschiedene Abstufungen zwischen grob und fein sind jeweils vorstellbar.

Chirurgische Feilen oder Raspeln, die hohen Belastungen ausgesetzt sind, sollten über eine adäquate Oberflächenhärte verfügen. Gleichwohl ist im Kern des jeweiligen Blattes eine hohe Zähigkeit gewünscht, um Brüche und dergleichen zu vermeiden.

Konventionelle chirurgische Feilen oder Raspeln nutzen daher häufig auch Hartmetallbacken oder Hartmetallblätter, die die gewünschten Härteeigenschaften mit sich bringen, aber als separate Bauteile mit dem stangenartigen, zähen Längsglied gefügt werden müssen.

Gemäß einer weiteren beispielhaften Ausgestaltung des Instruments oder des Verfahrens ist das stangenartig gestaltete Längsglied an seinem ersten Ende und an seinem zweiten Ende jeweils mit einem als Blatt gestalteten Trägerstück versehen, das einen strukturierten Kontaktabschnitt aufweist.

Die Strukturierung der beiden an voneinander abgewandten Enden platzierten Kontaktabschnitte kann sich voneinander unterscheiden. Auf diese Weise werden beispielsweise ein fein strukturierter Kontaktabschnitt und ein grob strukturierter Kontaktabschnitt miteinander kombiniert.

Es versteht sich, dass die anhand verschiedener Ausführungsbeispiele vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Offenbarung zu verlassen.

Weitere Merkmale und Vorteile der Offenbarung ergeben sich aus der nachfolgenden Beschreibung und Erläuterung mehrerer beispielhafter Ausführungsformen unter Bezugnahme auf die Zeichnungen. Es zeigen:
- Fig. 1:: eine Längsansicht einer Ausführungsform eines als Nadelhalter gestalteten medizinischen Instruments;
- Fig. 2:: eine gebrochene Längsansicht einer weiteren Ausführungsform eines als Nadelhalter gestalteten medizinischen Instruments;
- Fig. 3:: eine Längsansicht einer Ausführungsform eines als Pinzette gestalteten medizinischen Instruments;
- Fig. 4:: eine gebrochene Längsansicht einer Ausführungsform eines als Raspel oder Feile gestalteten medizinischen Instruments;
- Fig. 5:: eine schematische Detailansicht einer Ausführungsform eines strukturierten Kontaktabschnitts eines medizinischen Instruments;
- Fig. 6:: eine schematische Detailansicht einer weiteren Ausführungsform eines strukturierten Kontaktabschnitts eines medizinischen Instruments;
- Fig. 7:: eine schematische Detailansicht einer weiteren Ausführungsform eines strukturierten Kontaktabschnitts eines medizinischen Instruments;
- Fig. 8:: eine schematische Detailansicht einer weiteren Ausführungsform eines strukturierten Kontaktabschnitts eines medizinischen Instruments; und
- Fig. 9: ein vereinfachtes Blockdiagramm zur Veranschaulichung von Verfahrensschritten einer Ausführungsform eines Verfahrens zur Herstellung eines medizinischen Instruments.

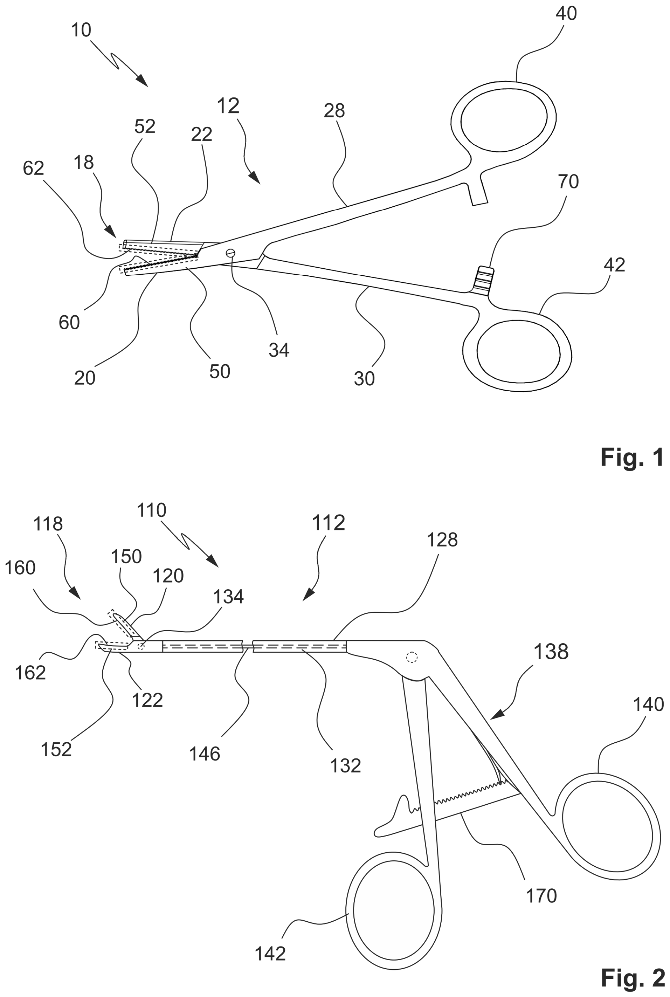

Fig. 1 zeigt ein insgesamt mit 10 bezeichnetes medizinisches Instrument, das im Ausführungsbeispiel als scherenartig gestalteter Nadelhalter 12 ausgebildet ist. Das Instrument 10 umfasst einen Maulkopf 18 mit einem ersten Maulteil 20 und einem zweiten Maulteil 22. Zwischen einem patientenfernen, proximalen Ende und einem patientennahen, distalen Ende erstrecken sich zwei Längsglieder 28, 30, die im Ausführungsbeispiel über ein Gelenk 34 gelenkig miteinander verbunden sind.

Der Maulkopf 18 mit den Maulteilen 20, 22 ist beim distalen Ende des Instruments 10 angeordnet. Beim proximalen Ende sind die Längsglieder 28, 30 jeweils mit einer Handhabe 40, 42 gekoppelt. Auf diese Weise kann ein Benutzer das Instrument 10 mit seiner Hand ergreifen und den Maulkopf 18 öffnen und schließen.

Im Ausführungsbeispiel weist das Längsglied 28 an seinem distalen Ende ein Trägerstück 50 auf. Das Längsglied 30 weist an seinem distalen Ende ein Trägerstück 52 auf. Die Trägerstücke 50, 52 weisen einander zugewandte Kontaktabschnitte 60, 62 auf, die mit einer Strukturierung (in Fig. 1 nicht detailliert dargestellt) versehen sind. Die Strukturierung umfasst beispielhaft eine Verzahnung, Riffelung oder dergleichen.

Offenbarungsgemäß sind die Trägerstücke 50, 52 mit den Kontaktabschnitten 60, 62 integral aus ein und demselben Grundwerkstoff gestaltet. Im Ausführungsbeispiel sind die Trägerstücke 50, 52 als Bestandteil der Maulteile 20, 22 integral mit den Längsgliedern 28, 30 gestaltet und aus ein und demselben Grundwerkstoff gebildet. Insbesondere handelt es sich bei dem Grundwerkstoff um einen korrosionsarmen oder korrosionsfreien Stahl, beispielsweise einen Edelstahl. Es sind jedoch offenbarungsgemäß festigkeitssteigernde Maßnahmen vorgesehen, um zumindest im Bereich der strukturierten Kontaktabschnitte 60, 62 die Oberfläche der Trägerstücke 50, 52 zu härten.

Auf diese Weise kann das als Nadelhalter 12 gestaltete Instrument 10 chirurgische Nadeln und Ähnliches fest und sicher greifen, ohne dass es zu übermäßigen Verschleiß der Kontaktabschnitte 60, 62 kommt. Zur Sicherung des geschlossenen Zustands des Maulkopfs 18 ist im Ausführungsbeispiel eine Arretierung 70 vorgesehen, die einen Rastmechanismus umfasst, der beim proximalen Ende der Längsglieder 28, 30 angeordnet ist.

Fig. 2 zeigt in ähnlicher Weise ein insgesamt mit 110 bezeichnetes medizinisches Instrument, welches im Ausführungsbeispiel als Nadelhalter 112 gestaltet ist. Der Nadelhalter 112 gemäß Fig. 2 eignet sich für endoskopische/laparoskopische Anwendungen. Das Instrument 110 umfasst einen Maulkopf 118. Ferner ist ein Längsglied 128 vorgesehen, das im Ausführungsbeispiel als Schaft 132 gestaltet ist. Das Längsglied 128 bzw. der Schaft 132 weisen eine ausgeprägte Längserstreckung auf, also ein großes Verhältnis zwischen Länge und Durchmesser. Die Darstellung in Fig. 2 ist entlang der Längserstreckung des Schafts 132 gebrochen. Das Instrument 110 kann folglich eine größere Längserstreckung aufweisen.

Beim proximalen Ende des Schafts 132 ist ein Griffabschnitt 138 angeordnet, der beispielsweise zwei Arme umfasst, die jeweils eine Handhabe 140, 142 ausbilden. Zumindest eine der Handhaben 140, 142 ist über ein Betätigungselement 146 mit dem Maulkopf 118 gekoppelt, um den Maulkopf 118 (mit zwei Maulteilen 120, 122) bedarfsweise zu öffnen und zu schließen. Bei dem Betätigungselement 146 handelt es sich beispielhaft um eine Stange oder einen Draht. Die beiden Maulteile 120, 122 können beispielhaft um ein Gelenk 134 relativ zueinander verschwenkt werden, um den Maulkopf 118 zu öffnen und zu schließen.

Beim Maulkopf 118 weist das Instrument 112 zwei Trägerstücke 150, 152 miteinander zugewandten strukturierten Kontaktabschnitten 160, 162 auf. Die Strukturierung umfasst beispielsweise eine Verzahnung, Riffelung oder dergleichen. Zur Sicherung des geschlossenen Zustands ist im Ausführungsbeispiel beim Griffabschnitt 138 eine Arretierung 170 vorgesehen, die einen Rastmechanismus aufweist.

Die Trägerstücke 150, 152 mit den Kontaktabschnitten 160, 162 sind jeweils integral mit einem der Maulteile 120, 122 gestaltet und aus jeweils ein und demselben Grundwerkstoff gebildet. Zur Härtesteigerung ist offenbarungsgemäß eine Oberflächenbehandlung mittels Niedertemperatur-Diffusionshärten zur Ausbildung einer oberflächennahen Diffusionszone vorgesehen, zumindest im Bereich der Strukturierung der Kontaktabschnitte 160, 162.

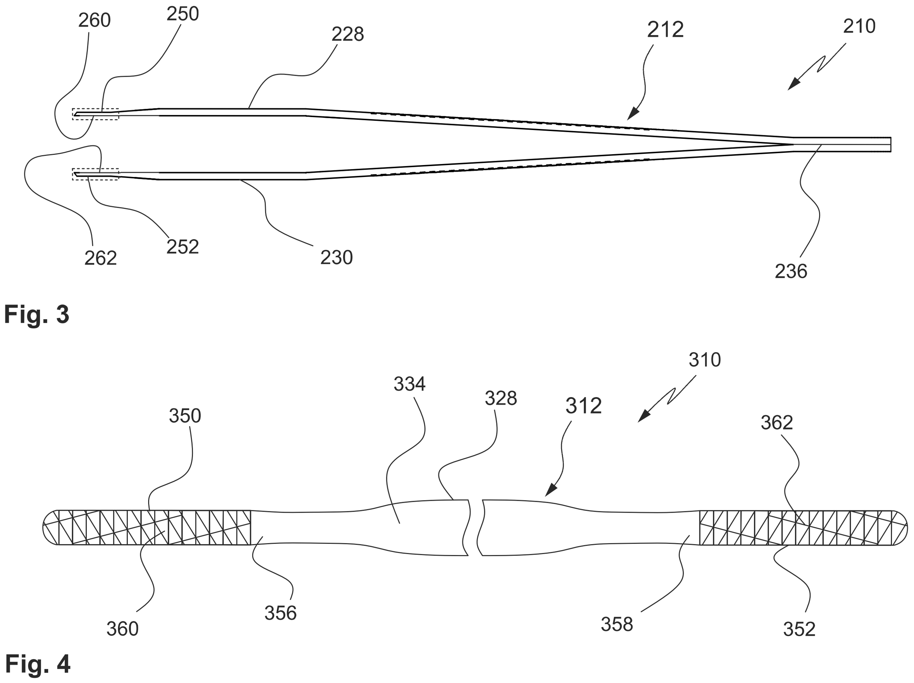

Fig. 3 veranschaulicht ein weiteres medizinisches Instrument 210 in Form einer Pinzette 212. Das Instrument 210 umfasst im Ausführungsbeispiel ein erstes Längsglied 228 und ein zweites Längsglied 230, die als Schenkel oder Branchen bezeichnet werden können. Die Längsglieder 228, 230 sind beim proximalen Ende des Instruments 210 miteinander verbunden und bilden dort eine Basis 236.

Das Längsglied 228 weist an seinem distalen Ende ein Trägerstück 250 mit einem strukturierten Kontaktabschnitt 260 auf. Das Längsglied 230 weist an seinem distalen Ende ein Trägerstück 252 mit einem strukturierten Kontaktabschnitt 262 auf. Die Kontaktabschnitte 260, 262 weisen jeweils eine Strukturierung auf, um das Ergreifen und Halten von Gewebe, Organen oder medizinischen Gerätschaften zu vereinfachen. Bei der Strukturierung handelt es sich beispielsweise um eine Verzahnung oder Riffelung. Die Strukturierung kann ähnlich einem Rändel oder Kreuzrändel gestaltet sein.

Die Längsglieder 228, 230 sind hinreichend elastisch gestaltet bzw. hinreichend elastisch miteinander gekoppelt, so dass die Kontaktabschnitte 260, 262 durch äußere Kraft auf das Instrument 210 zusammengedrückt werden können. Die Kontaktabschnitte 260, 262 weisen offenbarungsgemäß eine Oberflächenbehandlung mittels Niedertemperatur-Diffusionshärten zur Ausbildung einer oberflächennahen Diffusionszone auf. Die Trägerstücke 250, 252 mit den Kontaktabschnitten 260, 262 sind integral mit den Längsgliedern 228, 230 gestaltet, und zwar jeweils aus ein und demselben Grundwerkstoff. Trotz Härte steigernder Maßnahmen im Bereich der Kontaktabschnitte 260, 262 sind die Längsglieder 228, 230 hinreichend elastisch, so dass eine elastische Verformung zum Öffnen und Schließen des Instruments 210 ohne weiteres möglich ist.

Fig. 4 veranschaulicht ein Instrument 310, das als medizinische Raspel 312 gestaltet ist, insbesondere als sogenannte Knochenraspel. Alternativ kann das Instrument 310 als Feile oder Knochenfeile gestaltet sein.

Das Instrument 310 weist ein im Ausführungsbeispiel als langgestreckte Stange 334 gestaltetes Längsglied 328 auf (in Fig. 4 entlang der Längserstreckung gebrochen dargestellt). Das Längsglied 328 erstreckt sich zwischen einem ersten Ende und einem zweiten Ende. Bei jedem der beiden Enden ist ein Blatt 356, 358 ausgebildet, das ein Trägerstück 350, 352 ausbildet und trägt. Das Trägerstück 350 ist mit einem strukturierten Kontaktabschnitt 360 versehen. Das Trägerstück 352 ist mit einem strukturierten Kontaktabschnitt 362 versehen. Die Strukturierung umfasst jeweils eine Verzahnung mit Einzelzähnen oder Zahnreihen, eine Riffelung oder dergleichen.

Das Instrument 310 weist im Ausführungsbeispiel gemäß Fig. 4 an jedem seiner beiden Enden einen strukturierten Kontaktabschnitt 360, 362 auf. Demgemäß wird das Instrument 310 üblicherweise in einem mittigen Bereich durch einen Benutzer ergriffen und geführt. Es versteht sich, dass auch Gestaltungen von Knochenraspeln und ähnlichen medizinischen Feilen vorstellbar sind, bei denen nur ein Ende der beiden Enden mit einem strukturierten Kontaktabschnitt versehen ist.

Die Strukturierung der Kontaktabschnitte 360, 362 des Instruments 312 dient bewusst zum Materialabtrag. Daher ist auch hier eine offenbarungsgemäße Oberflächenbehandlung mittels Niedertemperatur-Diffusionshärten zur Ausbildung einer oberflächennahen Diffusionszone vorgesehen. Damit kann einerseits die Härte im oberflächennahen Bereich der strukturierten Kontaktabschnitte 360, 362 deutlich gesteigert werden. Gleichsam bleibt jedoch die Zähigkeit im Kern des jeweiligen Trägerstückes 350, 352 bzw. der Blätter 356, 358 der Stange 334 des Längsgliedes 328 erhalten.

Bei der Gestaltung des Instruments 310 gemäß Fig. 4 ist jeweils das aktuell dem Patienten zugewandte Ende (beispielsweise zum Abtrag organischen Materials wie Knochen oder Hartgewebe) das distale Ende. Das entsprechend vom Patienten abgewandte Ende ist das proximale Ende. Die Verwendung als distales oder proximales Ende kann sich entsprechend ändern.

Die Instrumente gemäß den Figuren 1-4 weisen im Bereich ihrer Kontaktabschnitte eine deutlich gesteigerte Härte auf, zumindest an der Oberfläche der jeweiligen Strukturierung. Diese Härtesteigerung kann unter Verzicht auf potenziell problematische Werkstoffe wie Hartmetalle und Ähnliches erzielt werden.

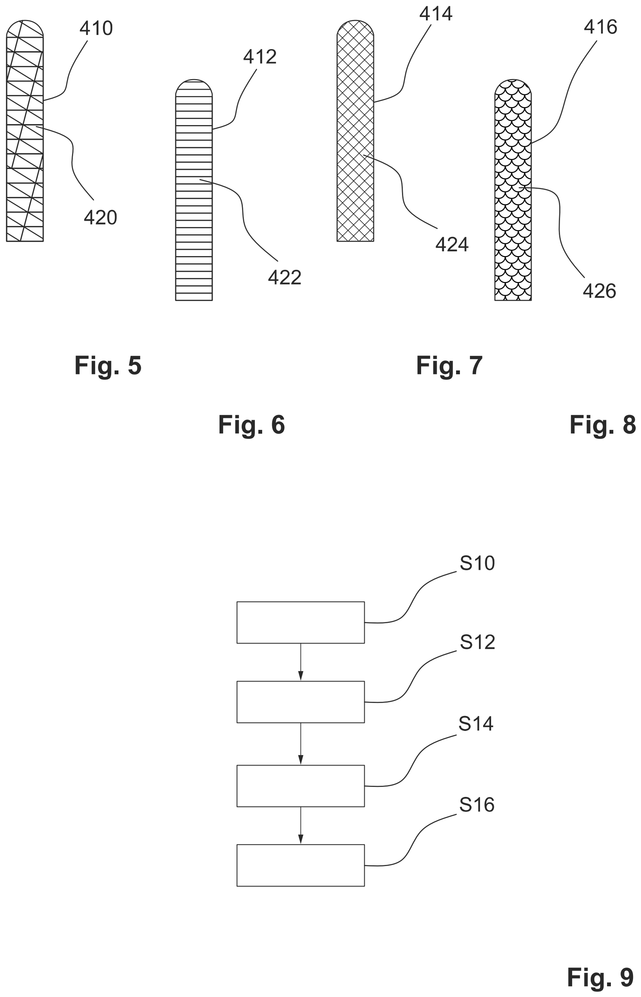

Die Figuren 5-8 zeigen beispielhafte Gestaltungen strukturierter Kontaktabschnitte für medizinische Instrumente. Fig. 5 zeigt ein Trägerstück 410 mit einem strukturierten Kontaktabschnitt 420, wobei die Strukturierung zwei oder mehr Vorzugsrichtungen aufweist. Fig. 6 zeigt ein Trägerstück 412 mit einem strukturierten Kontaktabschnitt 422, wobei die Strukturierung eine Vorzugsrichtung aufweist und ähnlich einem Rändel gestaltet ist. Fig. 7 zeigt ein Trägerstück 414 mit einem strukturierten Kontaktabschnitt 424, wobei die Strukturierung zwei Vorzugsrichtungen aufweist und ähnlich einem Kreuzrändel gestaltet ist. Fig. 8 zeigt ein Trägerstück 416 mit einem strukturierten Kontaktabschnitt 426, wobei die Strukturierung zueinander versetzte Reihen von Einzelzähnen aufweist. Sämtliche der strukturierten Kontaktabschnitte 420, 422, 424, 426 sind offenbarungsgemäß mit einer oberflächennahen Niedertemperatur-Diffusionshärtung versehen.

Fig. 9 veranschaulicht anhand eines vereinfachten Blockdiagramms eine beispielhafte Ausgestaltung eines Verfahrens zur Herstellung eines medizinischen Instruments, insbesondere eines chirurgischen oder endoskopischen Instruments.

Das Verfahren weist einen Schritt S10 auf, der die Bereitstellung eines Längsgliedes für ein Instrument umfasst, beispielsweise die Bereitstellung eines Arms oder eines Trägerschafts für ein Maulteilinstrument. Alternativ umfasst der Schritt S10 die Bereitstellung eines stangenartigen Längsgliedes für eine medizinische Feile oder Raspel.

Das Verfahren weist ferner einen Schritt S12 auf, der die Bereitstellung eines Trägerstückes aus einem korrosionsbeständigen Stahlwerkstoff, insbesondere aus einem Edelstahl umfasst. Das Trägerstück kann als integraler Bestandteil des Längsgliedes gestaltet sein. Es ist jedoch auch vorstellbar, das Trägerstück mit dem Längsglied zu koppeln. Das Trägerstück weist aufgrund des gewählten Werkstoffs einen hinreichend duktilen Kern, jedoch nur eine begrenzte Oberflächenhärte auf.

Das Verfahren weist ferner einen Schritt S14 auf, der die Erzeugung eines strukturierten Kontaktabschnitts auf dem Trägerstück umfasst, beispielsweise die Erzeugung einer Verzahnung oder einer Riffelung. Auf diese Weise kann die Strukturierung auf dem noch weichen Grundwerkstoff erzeugt werden. In bestimmten Ausgestaltungen ist der Schritt S14 zumindest dem Schritt S12 und gegebenenfalls dem Schritt S10 vorgelagert. Es sind jedoch auch Gestaltungen denkbar, bei denen der Schritt S14 dem Schritt S12 und gegebenenfalls dem Schritt S10 nachgelagert ist.

Das Verfahren weist einen Schritt S16 auf, der eine lokale Oberflächenbehandlung mittels Niedertemperatur-Diffusionshärten unter Ausbildung einer oberflächennahen Diffusionszone umfasst. Die Oberflächenbehandlung wird insbesondere im Bereich des strukturierten Kontaktabschnitts durchgeführt, um diesen zumindest oberflächlich zu Härten. Auf diese Weise kann insgesamt eine günstige Kombination aus harter Oberfläche und duktilem Kern bei den Trägerstücken und dem Instrument insgesamt erzielt werden.

### Beispiele

Trägerstücke in Form von Maulteilen aus ausgewählten korrosionsarmen Werkstoffen mit strukturierten Kontaktabschnitten wurden bereitgestellt. Die Maulteile wurden einer offenbarungsgemäßen Wärmebehandlung unterzogen.

Es ergaben sich für die gewählten Stichproben folgende gemittelten Messwerte für die resultierende Oberflächenhärte (HV 0.05) im Bereich der strukturierten Kontaktabschnitte:

**(Tabelle 1)**

| Werkstoff | Oberflächenhärte behandelt [HV 0.05] | Diffusionstiefe (µm) |
|---|---|---|
| 1.4307 | 905 | 26 |
| 1.4404 | 1150 | 35 |
| 1.4435 | 1008 | 38 |
| 1.4980 | 1100 | 31 |

Damit konnte eine deutliche Erhöhung der Oberflächenhärte erzielt werden, gerade im Vergleich zum unbehandelten Zustand. Vorzugsweise kann eine Oberflächenhärte erzielt werden, die der Härte von Hartmetallplatten bei konventionellen medizinischen Instrumenten vergleichbar ist. Die Diffusionstiefen wurden mittels metallografischer Untersuchungen optisch ermittelt.

Ergänzend wurde bei offenbarungsgemäß wärmebehandelten Exemplaren eine Härteverlaufsmessung zu Ermittlung der Diffusionstiefe in den gehärteten Bereichen durchgeführt. Es ergaben sich für die genannten Werkstoffe reproduzierbare Diffusionstiefen zwischen 25 µm und 40 µm, bei denen sich die Härte der Randzone der Grundwerkstoffhärte angleicht. In beispielhaften Ausgestaltungen wird eine Diffusionstiefe zwischen 15 µm und 40 µm angestrebt.

Daher ist auch bei den vergleichsweise geringen Querschnittsabmessungen offenbarungsgemäßer Instrumente gewährleistet, dass sich der Kern der Trägerstücke hinreichend duktil verhält. In bestimmten Ausgestaltungen beträgt der Durchmesser des Schaftes oder eines anderen Längsgliedes des Instruments weniger als 15 mm, in bestimmten Ausgestaltungen weniger als 12 mm, in bestimmten Ausgestaltungen weniger als 10 mm, in bestimmten Ausgestaltungen weniger als 8 mm, und in bestimmten Ausgestaltungen weniger als 6 mm. Da jedoch bewusst nur oberflächennahe Bereiche gehärtet werden, kann auch bei solchen vergleichsweise dünnen Werkstücken die Duktilität des Kerns gewahrt bleiben.

Insgesamt lässt sich auf diese Weise eine günstige Kombination aus harter Oberfläche im Bereich der Kontaktabschnitte und zähem, vergleichsweise elastischem Kern erzielen. Auf diese Weise lassen sich für verschiedene Anwendungen medizinischer Instrumente Hartmetalle substituieren. Wesentlich ist, dass die Oberflächenhärte in einem Maße gesteigert werden konnte, das die Substitution von Hartmetall-Plättchen erlaubt.

## Patentansprüche

1. Chirurgisches oder endoskopisches Instrument, mit zumindest einem Längsglied (28, 30; 128; 228, 230; 328) mit einer Längserstreckung zwischen einem ersten Ende und einem zweiten Ende, und einem Trägerstück (50, 52; 150, 152; 250, 252; 350, 352; 410; 412; 414; 416), das zumindest einen strukturierten Kontaktabschnitt (60, 62; 160, 162; 260, 262; 360, 362; 420; 422; 424; 426) aufweist, der insbesondere mit einer Verzahnung oder Riffelung versehen ist, wobei der zumindest eine strukturierte Kontaktabschnitt (60, 62; 160, 162; 260, 262; 360, 362; 420; 422; 424; 426) in das Trägerstück (50, 52; 150, 152; 250, 252; 350, 352; 410; 412; 414; 416) integriert ist, wobei das Trägerstück (50, 52; 150, 152; 250, 252; 350, 352; 410; 412; 414; 416) und der zumindest eine strukturierte Kontaktabschnitt (60, 62; 160, 162; 260, 262; 360, 362; 420; 422; 424; 426) aus einem korrosionsbeständigen Stahlwerkstoff bestehen, insbesondere aus einem Edelstahl, und wobei der zumindest eine strukturierte Kontaktabschnitt (60, 62; 160, 162; 260, 262; 360, 362; 420; 422; 424; 426) oberflächennah niedertemperatur-diffusionsgehärtet ist, insbesondere durch eine Oberflächenbehandlung mittels Niedertemperatur-Diffusionshärten unter Ausbildung einer oberflächennahen Diffusionszone.

2. Instrument nach Anspruch 1, wobei das Trägerstück (50, 52; 150, 152; 250, 252; 350, 352; 410; 412; 414; 416) und der zumindest eine strukturierte Kontaktabschnitt (60, 62; 160, 162; 260, 262; 360, 362; 420; 422; 424; 426) aus einem biokompatiblen Metallwerkstoff bestehen, insbesondere aus einem biokompatiblen Edelstahl.

3. Instrument nach Anspruch 1 oder 2, wobei das Instrument ausgewählt ist aus der Gruppe, die aus Folgendem besteht: Nadelhalter, medizinische Feilen, medizinische Raspeln und medizinische Pinzetten.

4. Instrument nach einem der Ansprüche 1 bis 3, wobei zumindest das Trägerstück (50, 52; 150, 152; 250, 252; 350, 352; 410; 412; 414; 416) und der zumindest eine strukturierte Kontaktabschnitt (60, 62; 160, 162; 260, 262; 360, 362; 420; 422; 424; 426), vorzugsweise das gesamte Instrument, hartmetallfrei gestaltet sind.

5. Instrument nach einem der Ansprüche 1 bis 4, wobei der zumindest eine strukturierte Kontaktabschnitt (60, 62; 160, 162; 260, 262; 360, 362; 420; 422; 424; 426) eine gesteigerte Oberflächenhärte aufweist.

6. Instrument nach einem der Ansprüche 1 bis 5, wobei der zumindest eine strukturierte Kontaktabschnitt (60, 62; 160, 162; 260, 262; 360, 362; 420; 422; 424; 426) eine Oberflächenhärte nach Vickers von zumindest 750 HV 0.05 aufweist, vorzugsweise von zumindest 850 HV 0.05, weiter bevorzugt von zumindest 950 HV 0.05, weiter bevorzugt von zumindest 1050 HV 0.05.

7. Instrument nach einem der Ansprüche 1 bis 6, wobei die Tiefe der Diffusionszone im strukturierten Kontaktabschnitt (60, 62; 160, 162; 260, 262; 360, 362; 420; 422; 424; 426) 10 µm bis 60 µm, vorzugsweise 25 µm bis 50 µm beträgt.

8. Instrument nach einem der Ansprüche 1 bis 7, wobei zumindest das Trägerstück (50, 52; 150, 152; 250, 252; 350, 352; 410; 412; 414; 416) einen duktilen Kern aufweist.

9. Instrument nach einem der Ansprüche 1 bis 8, wobei der korrosionsbeständige Werkstoff aus der Gruppe ausgewählt ist, die aus Folgendem besteht: nichtrostende, austenitische Chrom-Nickel-Stähle mit niedrigem Kohlenstoffgehalt, nichtrostende, austenitische Chrom-Nickel-Molybdän Edelstähle mit niedrigem Kohlenstoffgehalt, und korrosionsbeständige, warmfeste Eisen-Nickel-Chrom-Legierungen.

10. Instrument nach einem der Ansprüche 1 bis 9, wobei die Prozesstemperatur des Niedertemperatur-Diffusionsverfahrens unterhalb der Rekristallisationstemperatur des Stahlwerkstoffs gewählt ist.

11. Instrument nach einem der Ansprüche 1 bis 10, wobei das Instrument als Maulkopfinstrument gestaltet ist, insbesondere als Nadelhalter (12; 112), wobei zwei Maulteile (20, 22; 120, 122) vorgesehen sind, die relativ zueinander bewegbar sind, wobei jedes der zwei Maulteile (20, 22; 120, 122) ein Trägerstück (50, 52; 150, 152; 250, 252; 350, 352; 410; 412; 414; 416) ausbildet und mit einem strukturierten Kontaktabschnitt (60, 62; 160, 162; 260, 262; 360, 362; 420; 422; 424; 426) versehen ist, und wobei die strukturierten Kontaktabschnitte (60, 62; 160, 162; 260, 262; 360, 362; 420; 422; 424; 426) der zwei Maulteile (20, 22; 120, 122) zumindest im geschlossenen Zustand einander zugewandt sind.

12. Instrument nach Anspruch 11, wobei das Längsglied (28, 30; 128; 228, 230; 328) als Schaft (132) gestaltet ist, der sich zwischen einem proximalen Ende und einem distalen Ende erstreckt, wobei beim proximalen Ende ein Griffabschnitt (138) angeordnet ist, und wobei beim distalen Ende die zwei Maulteile (20, 22; 120, 122) angeordnet sind, von denen zumindest ein Maulteil (20; 120) relativ zum anderen Maulteil (22; 122) beweglich, insbesondere verschwenkbar, ist.

13. Instrument nach Anspruch 11 oder 12, wobei sich durch den Schaft (138) zumindest ein Betätigungselement (146) zur Betätigung des zumindest einen beweglichen Maulteils (20, 22; 120, 122) erstreckt.

14. Instrument nach einem der Ansprüche 11 bis 13, wobei eine Arretierung (70, 170) vorgesehen ist, die einen geschlossenen Zustand der zwei Maulteile (20, 22; 120, 122) sichert.

15. Instrument nach einem der Ansprüche 1 bis 10, wobei das Instrument als chirurgische Feile oder Raspel (310) gestaltet ist, wobei das Längsglied (28, 30; 128; 228, 230; 328) stangenartig gestaltet ist, wobei das zumindest eine Trägerstück (50, 52; 150, 152; 250, 252; 350, 352; 410; 412; 414; 416) an einem Blatt (356, 358) an einem ersten Ende oder zweiten Ende des Längsgliedes (28, 30; 128; 228, 230; 328) ausgebildet ist, und wobei das Blatt (356, 358) zumindest an einer Seite mit einem strukturierten Kontaktabschnitt (60, 62; 160, 162; 260, 262; 360, 362; 420; 422; 424; 426) versehen ist.

16. Instrument nach Anspruch 15, wobei das stangenartig gestaltete Längsglied (28, 30; 128; 228, 230; 328) an seinem ersten Ende und an seinem zweiten Ende jeweils mit einem ein Trägerstück (50, 52; 150, 152; 250, 252; 350, 352; 410; 412; 414; 416) aufweisenden Blatt (356, 358) versehen ist, das einen strukturierten Kontaktabschnitt (60, 62; 160, 162; 260, 262; 360, 362; 420; 422; 424; 426) aufweist.

17. Verfahren zur Herstellung eines chirurgischen oder endoskopischen Instruments, insbesondere nach einen der Ansprüche 1 bis 16, mit den folgenden Schritten:
- Bereitstellung zumindest eines Längsgliedes (28, 30; 128; 228, 230; 328) mit einer Längserstreckung zwischen einem ersten Ende und einem zweiten Ende,
- Bereitstellung eines Trägerstückes (50, 52; 150, 152; 250, 252; 350, 352; 410; 412; 414; 416) aus einem korrosionsbeständigen Stahlwerkstoff, insbesondere aus einem Edelstahl, wobei das Trägerstück (50, 52; 150, 152; 250, 252; 350, 352; 410; 412; 414; 416) als Abschnitt des zumindest einen Längsgliedes (28, 30; 128; 228, 230; 328) oder als mit dem Längsglied (28, 30; 128; 228, 230; 328) gekoppeltes Teil gestaltet ist,
- Erzeugung zumindest eines strukturierten Kontaktabschnitts (60, 62; 160, 162; 260, 262; 360, 362; 420; 422; 424; 426) auf dem Trägerstück (50, 52; 150, 152; 250, 252; 350, 352; 410; 412; 414; 416), insbesondere umfassend Erzeugung einer Verzahnung oder Riffelung,
- Durchführung einer lokalen Oberflächenbehandlung mittels Niedertemperatur-Diffusionshärten unter Ausbildung einer oberflächennahen Diffusionszone zum Härten des zumindest eines strukturierten Kontaktabschnitts (60, 62; 160, 162; 260, 262; 360, 362; 420; 422; 424; 426).
